# DEMANDE DE BREVET EUROPEEN

(11) **EP 3 610 940 A1**
(43) Date de publication de la demande: **19.02.2020**
(21) Numéro de dépôt: 19187228.2
(22) Date de dépôt: 19.07.2019
(51) Int. Cl.: B01D 53/22, B01D 53/30, C10L 3/10

(54) **TRAITEMENT PAR PERMÉATION MEMBRANAIRE AVEC AJUSTEMENT DE LA TEMPÉRATURE DU PREMIER RÉTENTAT EN FONCTION DE LA CONCENTRATION EN CH4 DANS LE TROISIÈME ET/OU QUATRIÈME PERMÉAT**

(30) Priorité: 08.08.2018 FR 1857383
(71) Demandeur: L'AIR LIQUIDE, SOCIETE ANONYME POUR L'ETUDE ET L'EXPLOITATION DES PROCEDES GEORGES CLAUDE, 75007 Paris (FR)
(72) Inventeur: CHAREYRE, Jean-Marc, 75007 PARIS (FR); GRABIE, Véronique, 75007 PARIS (FR); ZICK, Golo, 75007 PARIS (FR)
(74) Mandataire: Laigneau, Amandine

(57) **Abrégé**

Installation pour le traitement par perméation membranaire d'un flux gazeux d'alimentation comprenant au moins du méthane et du dioxyde de carbone, comprenant : un compresseur permettant de comprimer le flux gazeux d'alimentation, une première unité de séparation par membrane apte à recevoir le flux gazeux issu du compresseur et à fournir un premier perméat et un premier rétentat, une seconde unité de séparation par membrane apte à recevoir le premier rétentat et à fournir un second perméat et un second rétentat, une troisième unité de séparation par membrane apte à recevoir le premier perméat et à fournir un troisième perméat et un troisième rétentat, une quatrième unité de séparation par membrane apte à recevoir le troisième rétentat et à fournir un quatrième perméat et un quatrième rétentat, au moins un premier moyen de mesure de la concentration en CH4 dans le troisième perméat ou le quatrième perméat ou dans un flux combinant le troisième perméat ou le quatrième perméat, et au moins un moyen d'ajustement de la température du premier rétentat à l'entrée de la seconde unité de séparation par membrane en fonction de la mesure relevée par le premier moyen de mesure ; avec chaque unité de séparation par membrane comprenant au moins une membrane plus perméable au dioxyde de carbone qu'au méthane.

## Description

La présente invention est relative à une installation de traitement par perméation membranaire d'un courant gazeux contenant au moins du méthane et du dioxyde de carbone pour produire un courant gazeux riche en méthane - dont la teneur en méthane est conforme aux besoins de son utilisation et à un procédé de commande d'une telle installation.
Elle concerne en particulier l'épuration de biogaz, dans le but de produire du biométhane conforme aux spécifications pour injection dans un réseau de gaz naturel.
Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, appelé méthaniseur ou digesteur.
De par ses constituants principaux - méthane et dioxyde de carbone - le biogaz est un puissant gaz à effet de serre ; il constitue aussi, parallèlement, une source d'énergie renouvelable appréciable dans un contexte de raréfaction des énergies fossiles.
Le biogaz contient majoritairement du méthane (CH4) et du dioxyde de carbone (CO2) dans des proportions variables en fonction du mode d'obtention mais également, en moindres proportions de l'eau, de l'azote, de l'hydrogène sulfuré, de l'oxygène, ainsi que des composés organiques autres, à l'état de traces.

Selon les matières organiques dégradées et les techniques utilisées, les proportions des composants diffèrent, mais en moyenne le biogaz comporte, sur gaz sec, de 30 à 75% de méthane, de 15 à 60% de CO2, de 0 à 15% d'azote, de 0 à 5% d'oxygène et des composés traces.

Le biogaz est valorisé de différentes manières. Il peut, après un traitement léger, être valorisé à proximité du site de production pour fournir de la chaleur, de l'électricité ou un mélange des deux (la cogénération); la teneur importante en dioxyde de carbone réduit son pouvoir calorifique, augmente les coûts de compression et de transport et limite l'intérêt économique de sa valorisation à cette utilisation de proximité.
Une purification plus poussée du biogaz permet sa plus large utilisation, en particulier, une purification poussée du biogaz permet d'obtenir un biogaz épuré aux spécifications du gaz naturel et qui pourra lui être substitué ; le biogaz ainsi purifié est le « biométhane ». Le biométhane complète ainsi les ressources de gaz naturel avec une partie renouvelable produite au coeur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké sous forme de gaz naturel liquide (GNL)...

Les modes de valorisation du biométhane sont déterminés en fonction des contextes locaux : besoins énergétiques locaux, possibilités de valorisation en tant que biométhane carburant, existence à proximité de réseaux de distribution ou de transport de gaz naturel notamment. Créant des synergies entre les différents acteurs oeuvrant sur un territoire (agriculteurs, industriels, pouvoirs publics), la production de biométhane aide les territoires à acquérir une plus grande autonomie énergétique.
Plusieurs étapes doivent être franchies entre la collecte du biogaz et l'obtention du biométhane, produit final apte à être comprimé ou liquéfié.

En particulier, plusieurs étapes sont nécessaires avant le traitement qui vise à séparer le dioxyde de carbone pour produire un courant de méthane purifié. Une première étape consiste à comprimer le biogaz qui a été produit et acheminé à pression atmosphérique, cette compression peut être obtenue - de façon classique - via un compresseur. Les étapes suivantes visent à débarrasser le biogaz des composants corrosifs que sont le sulfure d'hydrogène et les composés organiques volatils (COV), les technologies utilisées sont de façon classique l'adsorption à pression modulée (PSA) et le piégeage sur charbon actif. Vient ensuite l'étape qui consiste à séparer le dioxyde de carbone pour disposer in fine de méthane à la pureté requise pour son usage ultérieur.

Le dioxyde de carbone est un contaminant typiquement présent dans le gaz naturel dont il est courant de devoir le débarrasser. Des technologies variées sont utilisées pour cela en fonction des situations ; parmi celles-ci, la technologie membranaire est particulièrement performante lorsque la teneur en CO2 est élevée ; elle est donc utilisée pour séparer le CO2 présent dans le biogaz, et en particulier dans le gaz de décharge. Les procédés membranaires de séparation de gaz utilisés pour la purification d'un gaz, qu'ils utilisent un ou plusieurs étages de membranes doivent permettre la production d'un gaz à la qualité requise, pour un faible coût, tout en minimisant les pertes du gaz que l'on souhaite valoriser. Ainsi, dans le cas de l'épuration du biogaz, la séparation effectuée est principalement une séparation CH4/CO2, devant permettre la production d'un gaz contenant en fonction de son utilisation plus de 85% de CH4, de préférence plus de 95% de CO2, plus préférentiellement plus de 97,5% de CH4, tout en minimisant les pertes de CH4 dans le gaz résiduaire et le coût d'épuration, ce dernier étant pour une part importante lié à la consommation électrique du dispositif de compression du gaz en amont des membranes.

Il est préférable que les installations permettant la production d'un flux gazeux enrichi en méthane puissent contrôler la perte de méthane.

Partant de là, un problème qui se pose est de fournir une installation permettant l'obtention d'un courant de méthane à concentration constante de manière à ce que les équipements qui utilisent le biomethane aient un fonctionnement régulier.

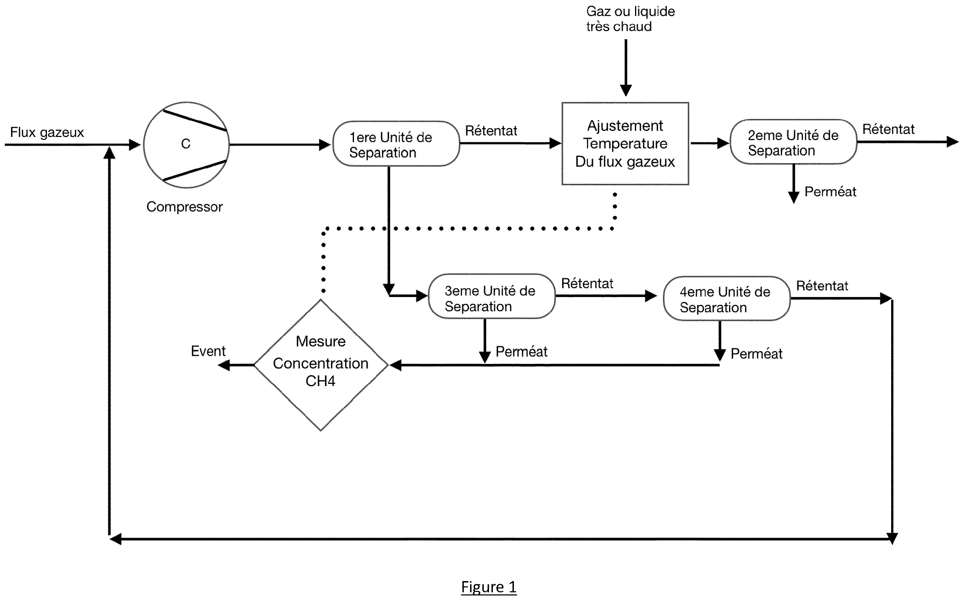

Une solution de la présente invention est une installation pour le traitement par perméation membranaire d'un flux gazeux d'alimentation comprenant au moins du méthane et du dioxyde de carbone, comprenant:
- un compresseur permettant de comprimer le flux gazeux d'alimentation,
- une première unité de séparation par membrane apte à recevoir le flux gazeux issu du compresseur et à fournir un premier perméat et un premier rétentat,
- une seconde unité de séparation par membrane apte à recevoir le premier rétentat et à fournir un second perméat et un second rétentat,
- une troisième unité de séparation par membrane apte à recevoir le premier perméat et à fournir un troisième perméat et un troisième rétentat,
- une quatrième unité de séparation par membrane apte à recevoir le troisième rétentat et à fournir un quatrième perméat et un quatrième rétentat,
- au moins un premier moyen de mesure de la concentration en CH4 dans le troisième perméat ou le quatrième perméat ou dans un flux combinant le troisième perméat ou le quatrième perméat, et
- au moins un moyen d'ajustement de la température du premier rétentat à l'entrée de la seconde unité de séparation par membrane en fonction de la mesure relevée par le premier moyen de mesure ;
avec chaque unité de séparation par membrane comprenant au moins une membrane plus perméable au dioxyde de carbone qu'au méthane.
Selon le cas l'installation selon l'invention peut présenter une ou plusieurs des caractéristiques suivantes :
- le premier moyen de mesure est effectué par un analyseur de gaz ;
- le moyen d'ajustement est un échangeur thermique mettant en oeuvre un flux secondaire présentant une température supérieure ou inférieure à la température du premier rétentat. Notons que ce flux secondaire peut être liquide ou gazeux. Sa température sera de préférence supérieure et sera de préférence comprise entre 1,2 fois la température du premier rétentat et 100 fois la température du premier rétentat, plus préférentiellement entre 2 fois la température du premier rétentat et 80 fois la température du premier rétentat, encore plus préférentiellement entre 2,5 fois la température du premier rétentat et 50 fois la température du premier rétentat ; La figure 1 illustre un exemple d'installation selon l'invention avec un flux secondaire de température supérieure à la température du premier rétentat ;
- le quatrième rétentat est recyclé au compresseur permettant de comprimer le flux gazeux d'alimentation.
- les membranes utilisées dans les unités de séparation par membrane ont la même sélectivité.
- au moins une unité de séparation par membrane comprend au moins deux membranes de même sélectivité ;
- au moins une unité de séparation par membrane comprend au moins deux membranes de sélectivités différentes
- au moins une unité de séparation par membrane utilise une membrane de sélectivité différente de la sélectivité des membranes des autres unités de séparation par membrane. La présente invention a également pour objet un procédé de commande d'une installation telle que définie dans l'invention, comprenant les étapes suivantes :
- une étape de mesure de la concentration en CH4 dans le troisième perméat ou le quatrième perméat ou dans un flux combinant le troisième perméat ou le quatrième perméat. La mesure sera effectuée de préférence par un analyseur de gaz ;
- une étape de comparaison de cette mesure avec une valeur consigne, et de détermination de l'écart par rapport à cette valeur consigne, et
- une étape d'ajustement de la température du premier rétentat à l'entrée de la seconde unité de séparation par membrane de manière à réduire l'écart déterminé.

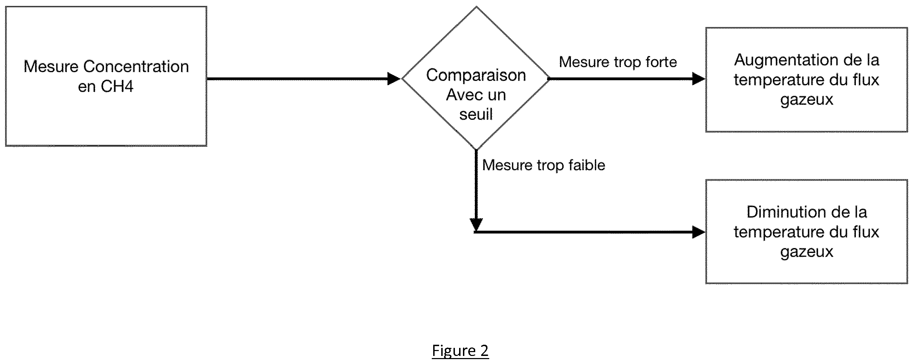

La figure 2 illustre le procédé selon l'invention.
Selon le cas, le procédé selon l'invention peut présenter une ou plusieurs des caractéristiques ci-dessous :
- l'ajustement de la température du premier rétentat est effectué par échange thermique entre un flux secondaire présentant une température supérieure ou inférieure à la température du premier rétentat. L'échange thermique est effectué au sein de l'échangeur thermique de l'installation selon l'invention ;
- l'étape d'ajustement comprend une augmentation ou une diminution de la température ;
- le flux gazeux d'alimentation est du biogaz.
- l'étape de comparaison et l'étape d'ajustement sont réalisées automatiquement par des moyens de transmission de données et de traitement de données. Un moyen de transmission de données et de traitement de données pourra être par exemple un calculateur industriel de type Automate Programmable.

## Revendications

1. Installation pour le traitement par perméation membranaire d'un flux gazeux d'alimentation comprenant au moins du méthane et du dioxyde de carbone, comprenant :
- un compresseur permettant de comprimer le flux gazeux d'alimentation,
- une première unité de séparation par membrane apte à recevoir le flux gazeux issu du compresseur et à fournir un premier perméat et un premier rétentat,
- une seconde unité de séparation par membrane apte à recevoir le premier rétentat et à fournir un second perméat et un second rétentat,
- une troisième unité de séparation par membrane apte à recevoir le premier perméat et à fournir un troisième perméat et un troisième rétentat,
- une quatrième unité de séparation par membrane apte à recevoir le troisième rétentat et à fournir un quatrième perméat et un quatrième rétentat,
- au moins un premier moyen de mesure de la concentration en CH4 dans le troisième perméat ou le quatrième perméat ou dans un flux combinant le troisième perméat ou le quatrième perméat, et
- au moins un moyen d'ajustement de la température du premier rétentat à l'entrée de la seconde unité de séparation par membrane en fonction de la mesure relevée par le premier moyen de mesure ;
avec chaque unité de séparation par membrane comprenant au moins une membrane plus perméable au dioxyde de carbone qu'au méthane.

2. Installation selon la revendication 1, **caractérisée en ce que** le premier moyen de mesure est effectué par un analyseur de gaz.

3. Installation selon l'une des revendications 1 ou 2, **caractérisée en ce que** le moyen d'ajustement est un échangeur thermique mettant en oeuvre un flux secondaire présentant une température supérieure ou inférieure à la température du premier rétentat.

4. Installation selon l'une des revendications 1 à 3, **caractérisée en ce que** le quatrième rétentat est recyclé au compresseur permettant de comprimer le flux gazeux d'alimentation.

5. Installation selon l'une des revendications 1 à 4, **caractérisée en ce que** les membranes utilisées dans les unités de séparation par membrane ont la même sélectivité.

6. Installation selon l'une des revendications 1 à 4, **caractérisée en ce que** au moins une unité de séparation par membrane utilise une membrane de sélectivité différente de la sélectivité des membranes des autres unités de séparation par membrane.

7. Installation selon l'une des revendications 1 à 6, **caractérisée en ce qu'**au moins une unité de séparation par membrane comprend au moins deux membranes de même sélectivité.

8. Installation selon l'une des revendications 1 à 6, **caractérisée en ce qu'**au moins une unité de séparation par membrane comprend au moins deux membranes de sélectivités différentes.

9. Procédé de commande d'une installation telle que définie dans l'une des revendications 1 à 8, comprenant les étapes suivantes :
- une étape de mesure de la concentration en CH4 dans le troisième perméat ou le quatrième perméat ou dans un flux combinant le troisième perméat ou le quatrième perméat. La mesure sera effectuée de préférence par un analyseur de gaz ;
- une étape de comparaison de cette mesure avec une valeur consigne, et de détermination de l'écart par rapport à cette valeur consigne, et
- une étape d'ajustement de la température du premier rétentat à l'entrée de la seconde unité de séparation par membrane de manière à réduire l'écart déterminé.

10. Procédé selon la revendication 8, **caractérisé en ce que** l'ajustement de la température du premier rétentat est effectué par échange thermique entre un flux secondaire présentant une température supérieure ou inférieure à la température du premier rétentat.

11. Procédé selon l'une des revendications 9 ou 10, **caractérisé en ce que** l'étape d'ajustement comprend une augmentation ou une diminution de la température.

12. Procédé selon l'une des revendications 9 à 11, **caractérisé en ce que** le flux gazeux d'alimentation est du biogaz.

13. Procédé selon l'une des revendications 9 à 12, **caractérisé en ce que** l'étape de comparaison et l'étape d'ajustement sont réalisées automatiquement par des moyens de transmission de données et de traitement de données.
